# EUROPEAN PATENT APPLICATION

(11) **EP 2 143 424 A1**
(43) Date of publication of application: **13.01.2010**
(21) Application number: 08738876.5
(22) Date of filing: 26.03.2008
(51) Int. Cl.: A61K 9/16, A61K 9/56, A61K 47/02, A61K 47/32, A61K 47/36, A61P 1/00, A61P 1/04, A61P 3/10, A61P 5/00, A61P 5/44, A61P 7/12, A61P 15/04, A61P 19/10, A61P 25/00, A61P 29/00, A61P 31/00, A61P 35/00, A61P 37/00, A61P 43/00

(54) **ORAL PHARMACEUTICAL PREPARATION FOR COLON-SPECIFIC DELIVERY**

(30) Priority: 26.03.2007 US 907230 P
(71) Applicant: TEIKOKU SEIYAKU CO., LTD., Higashikagawa-shi, Kagawa 769-2695 (JP)
(72) Inventor: KUBO, Hiroyuki, Yokohama-shi Kanagawa 224-0014 (JP)
(74) Representative: Ahner, Francis
(86) International application number: PCT/JP2008/055652
(87) International publication number: WO 2008/117814

(57) **Abstract**

The present invention relates to an oral pharmaceutical preparation having an excellent capability of delivering a drug to colon, more specifically an oral pharmaceutical preparation for delivering a drug to colon and comprising a core comprising at least a pharmaceutically acceptable vehicle, an inner layer covering said core and comprising said drug, an intermediate layer covering said inner layer and comprising a cationic polymer soluble or swellable at a pH of not more than 6.6, and an outer layer covering said intermediate layer and comprising an anionic polymer soluble at a pH of not less than 7.0.

## Description

### CROSS REFERENCE TO RELATED APPLICATION

This patent application claims priority based on U.S. Provisional Application 60/907,230 filed on March 26, 2007, the disclosure of which are herein incorporated by reference in its entirety.

### BACKGROUND OF THE INVENTION

### Field of the Invention

The present invention relates to an oral pharmaceutical preparation having a superior capability of delivering a drug to colon.

### Background Art

A rapid increase in the number of patients with inflammatory bowel disease has been recently reported. Inflammatory bowel disease is known as a chronic disease of unknown cause. Representative examples thereof include Crohn's disease and ulcerative colitis. From the viewpoint of improving QOL (Quality of life) of the patient with inflammatory bowel disease and the like, a pharmaceutical preparation for treating inflammatory bowel disease is hitherto studied. As the pharmaceutical preparation for treating inflammatory bowel disease, an oral pharmaceutical preparation and enema pharmaceutical preparation are mainly known and examples of a content drug thereof include steroid agents and the like.
Also, the large intestine has lower activities of digestive enzymes among the gastrointestinal tracts and is considered to be promising as an administration site for a peptide and/or protein aiming at systemic actions (Japanese Patent No. 3185206).

However, in the case of the oral pharmaceutical preparation in the prior art, the pharmaceutical preparation generally disintegrates in the stomach and small intestine and drug release is initiated. Therefore, when a drug is administrated to the large intestine, aiming at systemic actions, by the pharmaceutical preparation in the prior art, degradation of the drug by digestive enzymes occurs in the stomach, small intestine or the like and a desired amount of the drug is not absorbed from the gastrointestinal tract. As a result, it is difficult to deliver the drug in an amount enough to generate the systemic actions into the blood. On the other hand, in the case of a drug targeting a local region of the large intestine, including a therapeutic agent for inflammatory bowel disease, steroid agent or the like, the pharmaceutical preparation in the prior art may exhibit systemic side effects with the drug being absorbed while passing through the small intestine and delivered into systemic blood stream. Particularly when an inflammatory site subjected to be treated is located in the lower part of the gastrointestinal tract, drug release out of the pharmaceutical preparation begins in the gastrointestinal tract upper than the inflammatory site and the drug is absorbed in the small intestine or the like. As a result, a concentration of the drug at the inflammatory site is low and a sufficient pharmacological effect may not be attained.

In cases where the enema pharmaceutical preparation is used as a treatment for inflammatory bowel disease, burdens to the patient including discomfort by inserting a tube from the anus is problematic. Further, after administrated with the enema pharmaceutical preparation, the patient need to maintain a particular body posture such as a recumbent or prone posture for a certain period of time a body in order to have the drug widely distributed along the gastrointestinal tract wall and hence an administration mode of the enema pharmaceutical preparation is often complicated. Consequently, a pharmaceutical preparation overcoming these drawbacks of such an enema pharmaceutical preparation or the like, and allowing more convenient and effective drug administration has been desired.

As the pharmaceutical preparation described above, development of a pharmaceutical preparation for delivering to colon with a focus on an alteration of the environment (pH) inside the gastrointestinal tract has been examined. For instance, in Japanese Patent No.2821952, Japanese Patent No.2967492 and Japanese Patent No.3185206, an oral tablet for delivering to colon, which tablet can respond to the environment (pH) inside the gastrointestinal tract and release the drug after delivered to colon, is disclosed.

However, in the pharmaceutical preparation described above, it is difficult to attain a uniform coating thickness in the pharmaceutical preparation. Depending on a form of the pharmaceutical preparation, the pharmaceutical preparation may burst at a portion with thinner coating, and the drug may be released earlier than expected. When the coating thickness varies, by a physical force applied to the pharmaceutical preparation in the gastrointestinal tract, the pharmaceutical preparation may break before delivered to a targeted site in colon. In cases where the breakdown of the core containing the drug after the coating is dissolved is delayed, the pharmaceutical preparation may be discharged from the gastrointestinal tract before the drug is sufficiently released. As described above, the oral pharmaceutical preparation for delivering to colon in the prior art causes varied drug concentrations at the targeted site and fails to attain sufficient local concentration and absorption of the drug. Consequently, there are not few cases where intended therapeutic effects may not be attained. Accordingly, it can be said that an oral pharmaceutical preparation having superior abilities of delivering a drug to colon is still desired.

### SUMMARY OF THE INVENTION

The present inventors have now found a novel oral pharmaceutical preparation having an excellent capability of delivering a drug to colon. The present invention is based on this finding. Accordingly, an object of the present invention is to provide a novel oral pharmaceutical preparation having an excellent capability of delivering a drug to colon.

The oral pharmaceutical preparation for delivering the drug to colon according to the present invention comprises:
a core comprising at least a pharmaceutically acceptable vehicle;
an inner layer covering the core and comprising the drug;
an intermediate layer covering the inner layer and comprising a cationic polymer soluble or swellable at a pH of not more than 6.6; and
an outer layer covering the intermediate layer and comprising anionic polymer soluble at a pH of not less than 7.0.

The oral pharmaceutical preparation according to the present invention can respond to slight pH changes in the gastrointestinal tract to precisely release the drug in the large intestine. Accordingly, the oral pharmaceutical preparation according to the present invention can be advantageously used in that variation of a drug concentration in a targeted site in colon is suppressed and a sufficient therapeutic effect is attained.

### BRIEF DESCRIPTION OF THE DRAWINGS

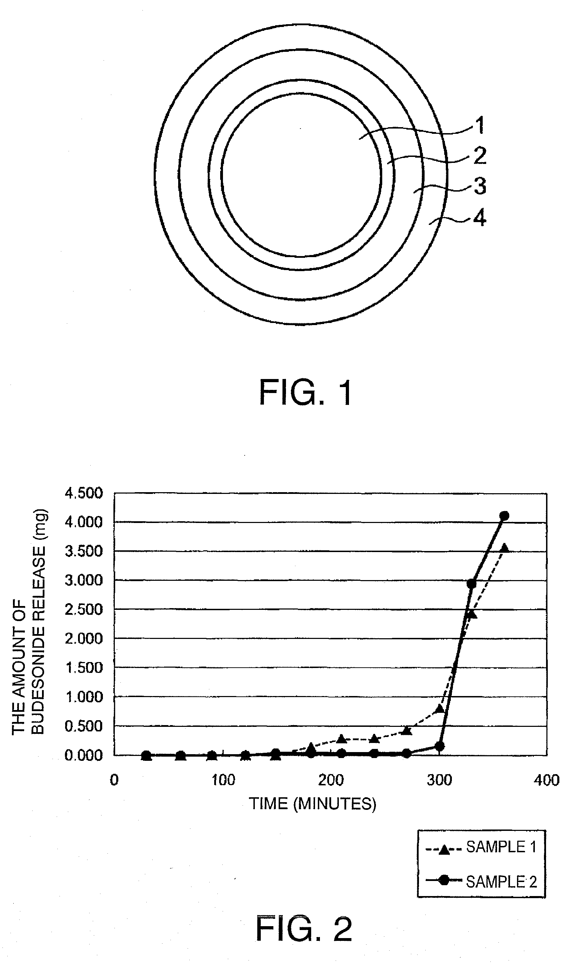

Figure1 is a cross-sectional view showing a mode of the oral pharmaceutical preparation according to the present invention.
Figure 2 is a graph showing the results of the dissolution test using the oral pharmaceutical preparation according to the present invention.

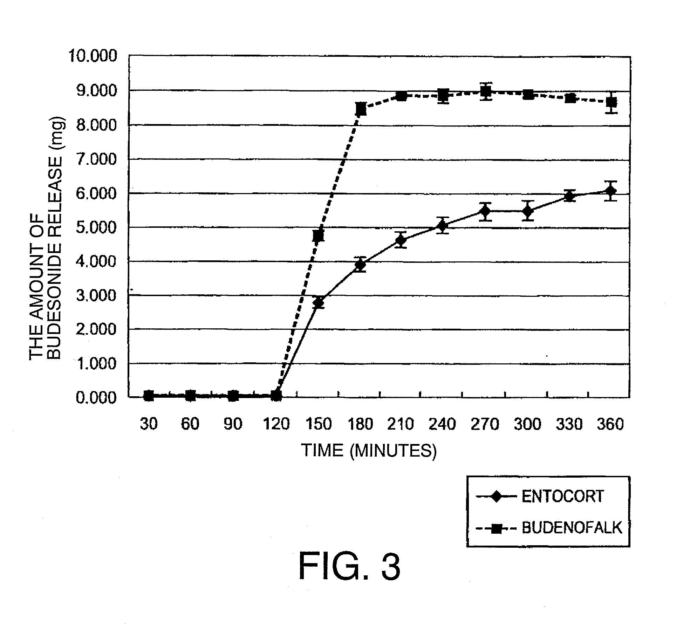

Figure 3 is a graph showing the results of the dissolution test using Entocort and Budenofalk.

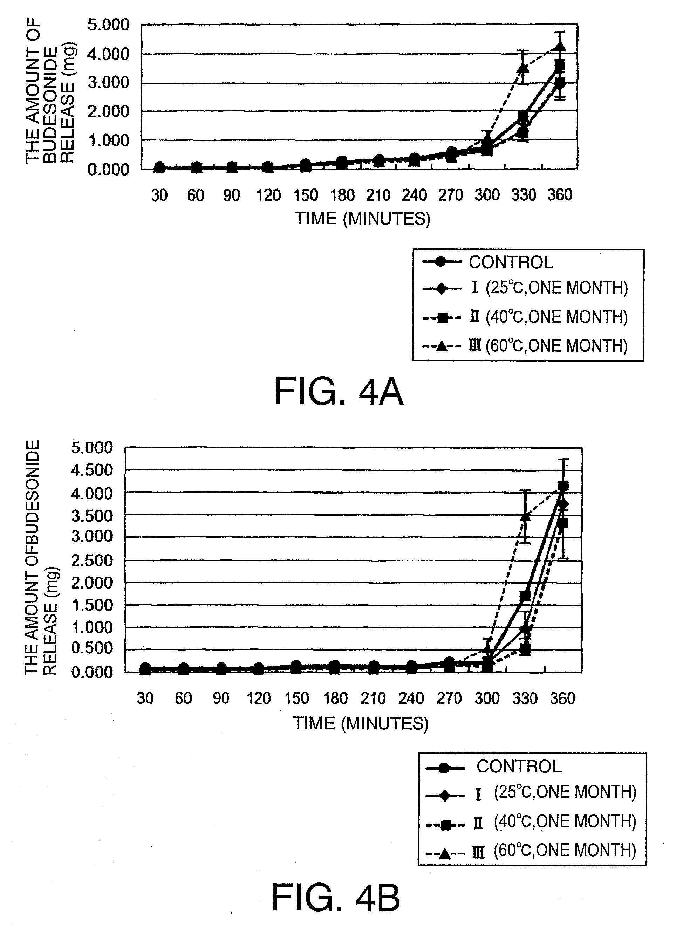

Figure 4A is a graph showing the results of the dissolution test using the oral pharmaceutical preparation according to the present invention (Sample 1: oral pharmaceutical preparation with the coating amount of the intermediate layer of 53.6% by weight and the coating amount of the outer layer of 32.3% by weight) one month after the start of the stability test.
Figure 4B is a graph showing the results of the dissolution test using the oral pharmaceutical preparation according to the present invention (Sample 2: oral pharmaceutical preparation with the coating amount of the intermediate layer of 53.6% by weight and the coating amount of the outer layer of 48.5% by weight) one month after the start of the stability test.

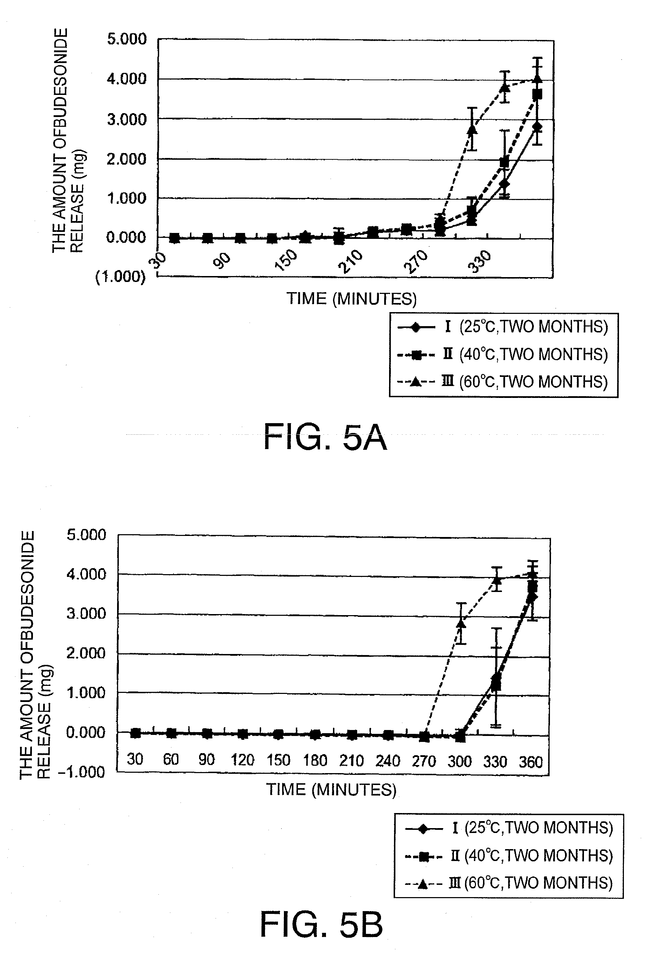

Figure 5A is a graph showing the results of the dissolution test using the oral pharmaceutical preparation according to the present invention (Sample 1: oral pharmaceutical preparation with the coating amount of the intermediate layer of 53.6% by weight and the coating amount of the outer layer of 32.3% by weight) two months after the start of the stability test.
Figure 5B is a graph showing the results of the dissolution test using the oral pharmaceutical preparation according to the present invention (Sample 2: oral pharmaceutical preparation with the coating amount of the intermediate layer of 53.6% by weight and the coating amount of the outer layer of 48.5% by weight) two months after the start of the stability test.

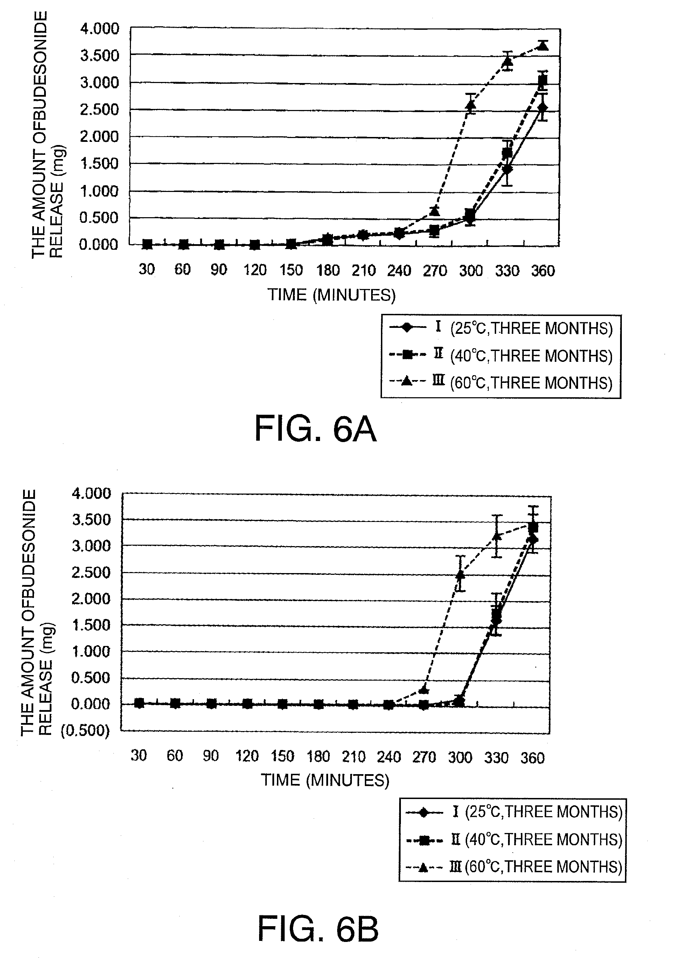

Figure 6A is a graph showing the results of the dissolution test using the oral pharmaceutical preparation according to the present invention (Sample 1: oral pharmaceutical preparation with the coating amount of the intermediate layer of 53.6% by weight and the coating amount of the outer layer of 32.3% by weight) three months after the start of the stability test.
Figure 6B is a graph showing the results of the dissolution test using the oral pharmaceutical preparation according to the present invention (Sample 2: oral pharmaceutical preparation with the coating amount of the intermediate layer of 53.6% by weight and the coating amount of the outer layer of 48.5% by weight) three months after the start of the stability test.

### DETAILED DESCRIPTION OF THE INVENTION

### Definition

The "alkyl" used herein means a linear, branched, or cyclic alkyl, preferably a linear alkyl.

### Oral pharmaceutical preparation

The oral pharmaceutical preparation according to the present invention is, as shown in Figure 1, **characterized in that** it has a laminated layer structure comprising (1) a core, (2) an inner layer comprising a drug, (3) an intermediate layer comprising a cationic polymer soluble or swellable at a pH of not more than 6.6, (4) an outer layer comprising anionic polymer soluble at a pH of not less than 7.0.

### Core

The core according to the present invention comprises at least a pharmaceutical acceptable vehicle. The above-described vehicle is preferably at least one selected from the group consisting of magnesium alumino silicate, calcium silicate, magnesium silicate, light anhydrous silicic acid, crystalline cellulose, crystalline cellulose·carmellose sodium, synthetic aluminum silicate, synthetic aluminum silicate·hydroxy propyl starch·crystalline cellulose, synthetic hydrotalcite, wheat flour, rice flour, rice starch, cellulose acetate phthalate, β-cyclodextrin, purified sucrose (preferably purified sucrose spherical granule or the like), low-substituted hydroxypropyl cellulose, dextran (preferably dextran 40 or the like), dextrin, natural aluminum silicate, corn starch (preferably corn starch granule or the like), lactose, lactose granule, saccharose, saccharose starch granule, potato starch, half-digested starch, microcrystalline cellulose, hydroxypropyl starch, hydroxypropyl cellulose, hydroxypropyl methylcellulose, hydroxypropyl methyl cellulose phthalate, partially-alphanized starch, powdered sugar and powdered cellulose; more preferably purified sucrose spherical granule, corn starch granule, lactose granule or saccharose starch granule; further preferably saccharose starch granule (Nonpareil (registered trademark), manufactured by Freund Corporation).

In addition, the above described core is preferably spherical. The spherical core is preferred from the viewpoint of making the thickness of coating of the inner, intermediate and outer layers uniform. Such uniform coating is advantageous in that it prevents the pharmaceutical preparation from bursting out of a thin coated portion and starting a drug release earlier than expected.

Furthermore, the average particle diameter of the above described core can be appropriately selected taking a size of the desired pharmaceutical preparation and the like into consideration, and is preferably 100 to 1000 µm, more preferably 180 to 850 µm, further preferably 350 to 850 µm.

### Inner layer

The inner layer according to the present invention is **characterized in that** it covers the above described core and comprises a drug. In the oral pharmaceutical preparation according to the present invention, the drug is uniformly dispersed around the core. Such a constitution is advantageous in that it prevents the drug release from delaying due to insufficient breakdown of the core.

In addition, the drug in the above described inner layer can be appropriately selected depending on a diseases subjected to be treated and examples thereof include a peptide pharmaceutical prone to be degraded in the stomach and/or small intestine, therapeutic agent for diabetes mellitus, therapeutic agent for osteoporosis, therapeutic agent for neurodegenerating diseases, therapeutic agent for endometriosis, promoting agent for uterine contraction and/or labor, ovulation inducing drug, therapeutic agent for acromegaly, agent for somatotropin substitution therapy, therapeutic agent for threatened abortion, therapeutic agent for diabetes insipidus, therapeutic agent for colorectal cancer, Lactobacillus pharmaceutical preparation, therapeutic agent for ulcerative colitis, therapeutic agent for immune abnormality diseases, therapeutic agent for inflammatory bowel disease such as therapeutic agent for Crohn's disease or therapeutic agent for irritable colon syndrome; preferably antitumor agent, antibiotic, polypeptide, anti-inflammatory agent, chemotherapeutic agent, immunosuppressant, steroid agent, vitamin, laxative, polynucleotide (gene pharmaceutical such as ribozyme, antisense, RNAi, decoy, aptamer or the like), Lactobacillus pharmaceutical preparation, therapeutic agent for ulcerative colitis, therapeutic agent for immune abnormality diseases, therapeutic agent for Crohn's disease or therapeutic agent for irritable colon syndrome; more preferably steroid agent.

More concretely, preferred examples of the above described drug include human insulin, peptide with human insulin-like action, thyroid hormone (PTH), peptide with PTH-like action, human calcitonin, peptide with human calcitonin-like action, thyrotropin releasing hormone (TRH), taltirelin hydrate, luteinizing hormone-releasing hormone (LH-RH), goserelin acetate, buserelin acetate, nafarelin acetate, oxitocin, human hypophyseal gonadotropin, octreotide acetate, somatropin, human chorionic gonadotropin, desmopressin acetate, 5-fluorouracil, bleomycin, doxifluridine, tegafur, tegafur 5-aminosalicylic acid, salazosulfapyridine, infliximab, budesonide, fluticasone propionate, beclometasone propionate ester, dexamethasone, dexamethasone acetate, dexamethasone palmitate, dexamethasone sodium metasulfobenzoate, dexamethasone sodium phosphate, triamcinolone, triamcinolone acetonide, hydrocortisone, hydrocortisone succinic acid ester sodium, fludrocortisone acetate, prednisolone, prednisolone succinic acid ester sodium , prednisolone sodium phosphate, beclometasone propionate ester, betamethasone, betamethasone d-chlorpheniramine maleic acid, betamethasone acetate·betamethasone sodium phosphate ester, betamethasone sodium phosphate ester, methylprednisolone, methylprednisolone sodium succinate or methylprednisolone acetate.

In addition, the above described inner layer preferably further comprises a coating agent. This coating agent can be appropriately selected, taking properties of the drug, a releasing rate thereof and the like into consideration, and is preferably a water soluble polymer, more preferably at least one selected from the group consisting of polyvinylpyrrolidone, gum arabic, hydroxypropylcellulose, hydroxypropyl methylcellulose, caramel, carboxymethyl ethyl cellulose, carboxymethyl starch sodium, carboxymethyl cellulose sodium, carboxymethyl cellulose calcium, triacetin, pullulan, propylene glycol, polyoxyethylene-polyoxypropylene glycol, polysorbate, polyvinyl alcohol, polyethylene glycol, mannitol and starch syrup; further preferably polyvinylpyrrolidone (polyvinylpyrrolidone K12, polyvinylpyrrolidone K15, polyvinylpyrrolidone K17, polyvinylpyrrolidone K25, Polyvinylpyrrolidone K30, polyvinylpyrrolidone K60, polyvinylpyrrolidone K90 or the like); further preferably polyvinylpyrrolidone K25.

Furthermore, the above described inner layer may contain a pharmaceutically acceptable additive such as plasticizer and binding inhibitor. The use of such an additive is preferred from the viewpoint of forming the uniform layer by coating.

Among the above described additive, examples of the plasticizer include polyethylene glycol, dioctyl adipate, adipic acid polyester, epoxidized soybean oil, epoxy hexahydro phthalate diester, triethyl citrate, glycerin, glycerine fatty acid ester, sesame oil, dimethylpolysiloxane silicon-dioxide mixture, D-sorbitol, medium chain fatty acid triglyceride, triacetin, sugar alcohol solution derived from corn starch, castor oil, phytosterol, diethyl phthalate, dioctyl phthalate, butyl phthalyl butyl glycolate, propylene glycol, polyoxyethylene-polyoxypropylene glycol, polysorbate, macrogol (macrogol 80, macrogol 400, macrogol 600, macrogol 1500, macrogol 4000, macrogol 6000 or the like), myristic acid isopropyl, cotton seed oil-soybean oil mixture, glyceryl monostearate or linoleic acid isopropyl.

Examples of the binding inhibitor include talc, hydrous silicon dioxide, light anhydrous silicic acid, crystalline cellulose, synthetic aluminum silicate, titanium oxide, heavy anhydrous silicic acid, magnesia alumina hydrate, stearic acid (calcium stearate, magnesium stearate or the like), tribasic calcium phosphate, corn starch, magnesium alumino metasilicate or dibasic calcium phosphate granule. The above-described additive can be appropriately used in the intermediate and outer layers, both of which are described below.

In addition, a coating amount of the inner layer can be appropriately determined, taking properties, dose of the drug and a quick drug release into consideration, and preferably 1 to 50% by weight of the weight of the above described core, more preferably 1 to 20% by weight, further preferably 5 to 10% by weight.

### Intermediate and outer layers

The intermediate and outer layers according to the present invention are constituted, as described below, taking prevention of a drug leakage at a pH of the stomach and small intestine and promotion of a drug release at a pH of the large intestine into consideration.

### Intermediate layer

The intermediate layer according to the present invention is **characterized in that** it covers the above described inner layer and comprises a cationic polymer soluble and swellable at a pH of not more than 6.6.
Such a cationic polymer is soluble and swellable at a pH of colon and enables the drug release in response to the pH of colon.

In a preferred mode according to the present invention, the cationic polymer in the above described intermediate layer can be soluble or swellable at a pH of not more than 6.4.
The above cationic polymer is preferably a copolymer composed of (meth)acrylic acid di C1-C2 alkyl amino C2-C4 alkyl and a monomer unit selected from (meth)acrylic acids C1-C4 alkyl, (meth)acrylic acids monohydroxy C2-C4 alkyl and a combination thereof, more preferably methyl (meth)acrylate·butyl (meth)acrylate·(meth)acrylic acid dimethylaminoethyl copolymer; further preferably methyl methacrylate·butyl methacrylate·methacrylic acid dimethylaminoethyl copolymer. This methyl methacrylate·butyl methacrylate·methacrylic acid dimethylaminoethyl copolymer is for example commercially available as EUDRAGIT E-100 (registered trademark) (Degussa Corporation, Germany, Dusseldorf).

Furthermore, the coating amount of the intermediate layer is, when conditions with a pH of not more than 6.6 continue, appropriately determined taking quick solubilization and swell of the intermediate layer into consideration, and preferably 15 to 75% by weight based on the total weight of the above described core and inner layer, preferably 30 to 60% by weight, further preferably 45 to 55% by weight.

### Outer layer

The outer layer according to the present invention is **characterized in that** it covers the above described intermediate layer and comprises an anionic polymer soluble at pH of not less than 7.0. Such an anionic polymer in the outer layer is not soluble at a normal pH of the stomach but is soluble at a pH of the small intestine. Thus the core covered with the intermediate layer can be protected by the outer layer until reaching colon.

The anionic polymer in the above described outer layer is preferably cellulose acetate phthalate, hydroxypropyl methylcellulose·phthalate, hydroxypropyl methylcellulose·acetate·succinate or (meth)acrylic acid·methyl (meth)acrylate copolymer, more preferably (meth)acrylic acid·methyl (meth)acrylate copolymer, more preferably methacrylic acid·methyl (meth)acrylate copolymer. This methacrylic acid·methyl (meth)acrylate copolymer is, for example, commercially available as EUDRAGIT S-100 (registered trademark) (Degussa Corporation, Germany, Dusseldorf).

In addition, the coating amount of the outer layer is, when conditions with a pH of not less than 7.0 continue, appropriately determined such that the outer layer is quickly solubilized, and preferably 15 to 70% by weight based on the total weight of the above described core and inner layer, more preferably 25 to 65% by weight, further preferably 30 to 50% by weight.

### Combination of cationic polymer and anionic polymer

According to a preferred embodiment of the present invention, the cationic polymer in the intermediate layer is the copolymer composed of (meth)acrylic acid di C1-C2 alkyl amino C2-C4 alkyl and a monomer unit selected from (meth)acrylic acid C1-C4 alkyl, (meth)acrylic acid monohydroxy C2-C4 alkyl and a combination thereof; and the anionic polymer in the outer layer is cellulose acetate phthalate, hydroxypropyl methylcellulose·phthalate, hydroxypropyl methylcellulose·acetate·succinate or (meth)acrylic acid·methyl (meth)acrylate copolymer.

According to a more preferred embodiment of the present invention, the cationic polymer in the intermediate layer is methyl (meth)acrylate·butyl (meth)acrylate·dimethylamino ethyl (meth)acrylate copolymer and the anionic polymer in the outer layer is (meth)acrylic acid·methyl (meth)acrylate copolymer.

According to a further preferred embodiment of the present invention, the cationic polymer in the intermediate layer is methyl methacrylate·butyl methacrylate·dimethylamino ethyl methacrylate copolymer and the anionic polymer in the outer layer is methacrylic acid·methyl (meth)acrylate copolymer.

### Form of the pharmaceutical preparation

The oral pharmaceutical preparation according to the present invention is the form of granules. The form of granules is advantageous from the viewpoint of reducing fluctuations of a disintegration time of the oral pharmaceutical preparation and releasing the drug precisely at a targeted site.
When the above described oral pharmaceutical preparation is a granule, the average particle size thereof can be appropriately selected, taking a desired timing and rate of the drug release into consideration, and can be, for example, 200 to 2000 µm.

### Production method

In the production of the oral pharmaceutical preparation according to the present invention, the core comprising the pharmaceutically acceptable vehicle is first prepared. This core may be produced by a known method such as mixing, granulation, drying, and particle sizing, and a commercially available spherical granule comprising the pharmaceutically acceptable vehicle may be used.

Subsequently, using a coating solution for the inner layer obtained by uniformly dispersing and dissolving the drug in conjunction with a coating agent and the like, in an aqueous medium, the core is coated and dried.
Examples of the aqueous medium include water, ethanol or a mixed solution thereof. This aqueous medium can be preferably used in coating of the intermediate and outer layers described below as well.

Next, using a coating solution for the intermediate layer obtained by dissolving the cationic polymer soluble and swellable at a pH of not more than 6.6 in the aqueous medium, the above described core covered with the inner layer is further coated and dried.

Thereafter, using a coating solution for the outer layer obtained by dissolving the anionic polymer soluble at a pH of not less than 7.0 in the aqueous medium, the above described core covered with the intermediate layer and inner layer is coated and further dried. The oral pharmaceutical preparation according to the present invention can be thereby obtained.
Conditions for coating and drying in the above described method can be appropriately determined by those skilled in the art, depending on the drug and properties of the polymer and coating agent used, and the like.

### Application

The oral pharmaceutical preparation according to the present invention has excellent capabilities of delivering the drug to the large intestine and is advantageous in terms of selectively delivering the drug depending on a targeted site in colon. Accordingly, the oral pharmaceutical preparation according to the present invention is preferably used in order to deliver the drug to the ascending colon, transverse or descending colon.

In addition, a disease subjected to be treated with the oral pharmaceutical preparation according to the present invention can be appropriately determined depending on properties of the drug to be selected. Examples of such a disease subjected to be treated include inflammatory bowel disease and the like, preferably diabetes mellitus, osteoporosis, neurodegenerating disease, endometriosis, inertia of uterine contraction and labor, amenorrhea, acromegaly, deficiency of growth hormone, threatened abortion, diabetes insipidus, colorectal cancer, decreased large intestine function due to decrease in the number or function of Lactobacillus, ulcerative colitis, immune abnormality disease, inflammatory disease such as Crohn's disease, or irritable colon syndrome; more preferably ulcerative colitis, immune abnormality disease, Crohn's disease, or irritable colon syndrome.

### EXAMPLES

The present invention will now be described more concretely by way of an example thereof. However, the present invention is not restricted to these Examples.

### Production of Oral Pharmaceutical Preparation 1

As the core of oral pharmaceutical preparation, 500 g of Nonpareil 101 (24/32) (manufactured by Freund Corporation) with a particle diameter of 500 to 710 µm was selected.
Next, a coating solution for the inner layer, which solution is prescribed below, was prepared.

| | |
|---|---|
| Budesonide (manufactured by Crystal Parma (Italy)) | 1.16 parts by weight |
| PVP K25 (manufactured by Wako Pure Chemical) | 1.16 parts by weight |
| Talc (manufactured by Wako Pure Chemical) | 4.65 parts by weight |
| Water Pharmacopoeia ethanol mixed solution (water/Pharmacopoeia ethanol = 2/8) | 93 parts by weight |

The above described coating solution was applied to the core with a coating machine (Granurex GX-20, manufactured by Freund Corporation) under predetermined conditions (revolution 400 rpm, slit air amount 0.3 m³/min, exhaust air amount 0.4 m³/min, supply air temperature 40°C, exhaust air temperature 40°C, spray air pressure 0.15 MPa). An increase in weight by this coating was 60 g, which was equivalent to 12% by weight of the weight of the core prior to coating. The thus obtained core covered with the inner layer was dried and used in the procedure below.

A coating solution for the intermediate layer with the following prescription was prepared.

| | |
|---|---|
| EUDRAGIT S-100 (Degussa Corporation) parts by weight | 7 by weight |
| Ethanol | 70 parts by weight |
| Water | 19.5 parts by weight |
| Talc | 3.5 parts by weight |

The above described coating solution for the intermediate layer was applied to 500 g of the core covered with the inner layer using the coating machine (Granurex GX-20, manufactured by Freund Corporation) under the predetermined conditions (revolution 400 rpm, slit air amount 0.3 m³/min, exhaust air amount 0.4 m³/min, supply air temperature 45°C, exhaust air temperature 45°C, spray air pressure 0.15 MPa). An increase in the weight of the core was 267.82 g. The weight increase by this coating was equivalent to 53.6% by weight of the total weight of the core and inner layer. The thus obtained core covered with the intermediate and inner layers was dried and used in the procedure below.

A coating solution for the outer layer with the following prescription was prepared.

| | |
|---|---|
| EUDRAGIT S-100 (Degussa Corporation) | 7 parts by weight |
| Ethanol | 70 parts by weight |
| Water | 18.8 parts by weight |
| Talc | 3.5 parts by weight |
| Polyethylene glycol 6000 | 0.7 parts by weight |

The above described coating solution for the intermediate layer was applied to 500 g of the core covered with the intermediate and inner layers using the coating machine (Granurex GX-20, manufactured by Freund Corporation) under the predetermined conditions (revolution 400 rpm, slit air amount 0.3 m³/min, exhaust air amount 0.4 m³/min, supply air temperature 45°C, exhaust air temperature 45°C, spray air pressure 0.15 MPa). An increase in weight by this coating was 161.34 g, which was equivalent to 32.3% by weight of the total weight of the core, inner layer and intermediate layer. The thus obtained core covered with the outer, intermediate and inner layers was dried to obtain an oral pharmaceutical preparation (hereinafter referred to as "Sample 1")

### Production of Oral Pharmaceutical Preparation 2

By the same production method as in the above described Sample 1, Samples 2 to 9 having varied coating amount of the intermediate and outer layers were prepared. The coating weight of the intermediate and outer layers in the oral pharmaceutical preparations including the Sample 1 is shown in Table 1.

**[Table 1]**

| Sample No. | The coating amount of the intermediate layer^{*1} | The coating amount of the outer layer^{*2} |
|---|---|---|
| 1 | 53.6% by weight | 32.3% by weight |
| 2 | 53.6% by weight | 48.5% by weight |
| 3 | 53.6% by weight | 64.5% by weight |
| 4 | 17.9% by weight | 32.3% by weight |
| 5 | 17.9% by weight | 48.5% by weight |
| 6 | 35.7% by weight | 32.3% by weight |
| 7 | 35.7% by weight | 48.5% by weight |
| 8 | 71.4% by weight | 48.5% by weight |
| 9 | 71.4% by weight | 64.5% by weight |

| | | |
|---|---|---|
| *1: The coating amount of the intermediate layer (% by weight) is a value calculated based on the total weight of the core and inner layer. *2: The coating amount of the outer layer (% by weight) is a value calculated based on the total weight of the core, inner layer and intermediate layer. | | |

### Dissolution Test

For the oral pharmaceutical preparation of the Samples 1 and 2, a dissolution test was carried out under the following condition. In addition, as a reference example, the dissolution test was also carried out for Entocort (registered trademark) (AstraZeneca) and Budenofalk (registered trademark) (Dr. Falk Pharma), both of which were a commercially available pharmaceutical preparation.

### Samples 1 and 2

As for the Samples 1 and 2, test solutions with pHs of 1.2, 7.4 and 6.4 were used and rotary basket method was applied.
The oral medicinal preparation was first placed 1 g each in each basket. Then, Japanese Pharmacopoeia test solution with a pH of 1.2 (900 ml) equivalent to gastric juice in the stomach was poured in a container for test solution to check a release of the drug for 120 minutes. In this case, the temperature was 37 °C and the revolution was 200 rpm. At a time point of 30, 60, 90 and 120 minutes, 5mL each was sampled. After sampling, 5 ml of Japanese Pharmacopoeia test solution with a pH of 1.2 was supplemented. An amount of the drug in each sampled solution was checked by UPLC (manufactured by Nihon Waters K.K.). In addition, when air bubbles were involved at the lower portion of the basket, the air bubbles were removed with a spatula.

Next, the test solution was exchanged with a solution with a pH of 7.4 which is equivalent to the pH of the small intestine (adjusted with Mcllvaine's buffer solution; 900 ml; 50 mM disodium hydrogen phosphate, 25 mM citric acid) and an amount of the drug release for 120 minutes was further checked by the same sampling manner as described above.

Subsequently, the test solution was exchanged with a final solution with a pH of 6.4 which is equivalent to the pH of the large intestine (adjusted with Mcllvaine's buffer solution; 900mL; 50 mM disodium hydrogen phosphate, 25 mM citric acid) and an amount of drug release for 120 minutes was further checked by the same sampling manner as described above.

The results of the dissolution test of the Samples 1 and 2 were shown in Figure 2. Budesonide release from the Samples 1 and 2 was not observed at a pH of 1.2 whereas the amount of budesonide release was not more than 0.3 mg at a pH of 7.4. On the other hand, the amount of budesonide release increased to not less than 3.0 mg at a pH of 6.4 for 120 minutes.

### Reference Example

For Entocort and Budenofalk, which are commercially available pharmaceutical preparations, the rotary basket method was applied using test solutions with pHs of 1.2 and 7.4. In this case, using three capsules of each commercially available pharmaceutical preparation, the dissolution test was carried out for 120 minutes for the test solution with a pH of 1.2 and 240 minutes for the test solution with a pH of 7.4. Sampling was carried out every 30 minutes.

The results of the dissolution test of Entocort and Budenofalk were shown in Figure 3. In both Entocort and Budenofalk, budesonide release was not observed at a pH of 1.2. On the other hand, at a pH of 7.4, the amount of budesonide release of Entocort increased to 6 mg for 240 minutes whereas the amount of budesonide release of Budenofalk increased to 9 mg for 240 minutes.
In Entocort and Budenofalk, it was observed that dissolution did not substantially occur at a pH equivalent to the pH of the stomach (pH1.2) whereas budesonide was released at a pH equivalent to the pH of the small intestine (pH7.4).

### Stability Test

The Samples 1 and 2 were placed in a thermo-hygrostat which was set in predetermined storage conditions, and taken out one, two and three months later, to be subjected to the dissolution test. As the above described storage conditions, I (25°C, 60% RH), II (40°C, 60% RH) and III (60°C, 75% RH) were selected.

As for the Sample 1, the results of the dissolution test one month after the stability start of the test were shown in Figure 4A. As for the Sample 2, the results of the dissolution test one month after the start of the stability test were shown in Figure 4B. Under all of the storage conditions (I, II, III), the amount of budesonide release of the Samples 1 and 2 was not more than 0.3 mg at both pHs of 1.2 and 7.4. On the other hand, under all of the storage conditions, at a pH of 6.4, the amount of budesonide release of the Samples 1 and 2 increased to not less than 3.5 mg for 120 minutes.

As for the Sample 1, the results of the dissolution test two months after the stability start of the test were shown in Figure 5A. As for the Sample 2, the results of the dissolution test three months after the start of the stability test were shown in Figure 5B. Under all of the storage conditions (I, II, III), the amount of budesonide release of the Samples 1 and 2 was not more than 0.2 mg at both pHs of 1.2 and 7.4. On the other hand, under all of the storage conditions, at a pH of 6.4, the amount of budesonide release of the Samples 1 and 2 increased to not less than 2.5 mg for 120 minutes.

As for the Sample 1, the results of the dissolution test three months after the start of the stability test were shown in Figure 6A. As for the Sample 2, the results of the dissolution test three months after the stability start of the test were shown in Figure 6B. Under all of the storage conditions (I, II, III), the amount of budesonide release of the Samples 1 and 2 was not more than 0.25 mg at both pHs of 1.2 and 7.4. On the other hand, under all of the storage conditions, at a pH of 6.4, the amount of budesonide release of the Samples 1 and 2 increased to not less than 2.5 mg for 120 minutes.

As shown in Figures 4 to 6, under any of the conditions of I (25°C, 60% RH), II (40°C, 60% RH) and III (60°C, 75% RH), a tendency that the dissolution did not occur at a pH equivalent to the pH of the stomach and small intestine whereas the drug release occurred at a pH equivalent to the pH of the large intestine was observed.

## Claims

1. An oral pharmaceutical preparation for delivering a drug to colon, said preparation comprising:
a core comprising at least a pharmaceutically acceptable vehicle;
an inner layer covering said core and comprising said drug;
an intermediate layer covering said inner layer and comprising a cationic polymer soluble or swellable at a pH of not more than 6.6; and
an outer layer covering said intermediate layer and comprising an anionic polymer soluble at a pH of not less than 7.0.

2. The oral pharmaceutical preparation according to claim 1, wherein said vehicle is at least one selected from the group consisting of magnesium alumino silicate, calcium silicate, magnesium silicate, light anhydrous silicic acid, crystalline cellulose, crystalline cellulose·carmellose sodium, synthetic aluminum silicate, synthetic aluminum silicate.hydroxypropyl starch·crystalline cellulose, synthetic hydrotalcite, wheat flour, rice flour, rice starch, cellulose acetate phthalate, β-cyclodextrin, purified sucrose, low-substituted hydroxypropyl cellulose, dextran, dextrin, natural aluminum silicate, corn starch, lactose, lactose granule, saccharose, saccharose starch granule, potato starch, half-digested starch, microcrystalline cellulose, hydroxypropyl starch, hydroxypropyl cellulose, hydroxypropyl methyl cellulose, hydroxypropyl methyl cellulose phthalate, partially-alphanized starch, powdered sugar and powdered cellulose.

3. The oral pharmaceutical preparation according to claim 1, wherein said core is spherical.

4. The oral pharmaceutical preparation according to claim 3, wherein the average particle diameter of said core is 100 to 1000 µm.

5. The oral pharmaceutical preparation according to claim 1, wherein said drug is anti-tumor agent, antibiotic, polypeptide, anti-inflammatory agent, chemotherapeutic agent, immnosuppressant, steroid agent, vitamin, laxative, polynucleotide, Lactobacillus preparation, therapeutic agent for ulcerative colitis, therapeutic agent for immune abnormality disease, therapeutic agent for Crohn's disease, therapeutic agent for irritable colon syndrome.

6. The oral pharmaceutical preparation according to claim 5, wherein said drug is said steroid agent.

7. The oral pharmaceutical preparation according to claim 1, wherein said inner layer further comprises a water soluble polymer.

8. The oral pharmaceutical preparation according to claim 7, wherein said water soluble polymer is at least one selected from the group consisting of polyvinylpyrrolidone, gum arabic, hydroxypropyl cellulose, hydroxypropyl methylcellulose, caramel, carboxymethyl ethyl cellulose, carboxymethyl starch sodium, carboxymethyl cellulose sodium, carboxymethyl cellulose calcium, triacetin, pullulan, propylene glycol, polyoxyethylene-polyoxypropylene glycol, polysorbate, polyvinyl alcohol, polyethylene glycol, mannitol and starch syrup.

9. The oral pharmaceutical preparation according to claim 1, wherein the coating amount of said inner layer is 1 to 50% by weight of said core.

10. The oral pharmaceutical preparation according to claim 1, wherein said cationic polymer is a copolymer composed of (meth)acrylic acid di C1-C2 alkyl amino C2-C4 alkyl and a monomer unit selected from (meth)acrylic acid C1-C4 alkyl, (meth)acrylic acid monohydroxy C2-C4 alkyl and a combination thereof.

11. The oral pharmaceutical preparation according to claim 10, wherein said cationic polymer of the intermediate layer is methyl (meth)acrylate·butyl (meth)acrylate·(meth)acrylic acid dimethylaminoethyl copolymer.

12. The oral pharmaceutical preparation according to claim 1, wherein the coating amount of the intermediate layer is 15 to 75% by weight based on the total weight of said core and inner layer.

13. The oral pharmaceutical preparation according to claim 1, wherein said anionic polymer of the outer layer is cellulose acetate phthalate, hydroxypropyl methyl cellulose phthalate, hydroxypropyl methyl cellulose·acetate·succinate or (meth)acrylic acid·methyl (meth)acrylate copolymer.

14. The oral pharmaceutical preparation according to claim 13, wherein said anionic polymer of the outer layer is (meth)acrylic acid·methyl (meth)acrylate copolymer.

15. The oral pharmaceutical preparation according to claim 1, wherein the coating amount of the outer layer is 15 to 70% by weight based on the total weight of said core, inner layer and intermediate layer.

16. The oral pharmaceutical preparation according to claim 1 which is a granule.

17. The oral pharmaceutical preparation according to claim 1 for delivering said drug to the ascending colon, transverse colon or descending colon.

18. The oral pharmaceutical preparation according to claim 5 for treating diabetes mellitus, osteoporosis, neurodegenerating disease, endometriosis, inertia of uterine contraction and labor, amenorrhea, acromegaly, deficiency of growth hormone, threatened abortion, diabetes insipidus, colorectal cancer, decreased large intestine function due to decrease in the number or function of Lactobacillus, ulcerative colitis, immune abnormality disease, Crohn's disease, or irritable colon syndrome.
